# EUROPEAN PATENT APPLICATION

(11) **EP 1 374 832 A1**
(43) Date of publication of application: **02.01.2004**
(21) Application number: 02703941.1
(22) Date of filing: 08.03.2002
(51) Int. Cl.: A61K 7/00

(54) **COSMETICS TO PROMOTE MATURATION OF CORNIFIED ENVELOPE AND METHOD FOR TREATMENT**

(30) Priority: 13.03.2001 JP 2001070263
(71) Applicant: SHISEIDO COMPANY, LTD., Tokyo 104-8010 (JP)
(72) Inventor: HIRAO, Tetsuji, Shiseido Res. Ctr, Yokohama-shi, Kanagawa 236-8643 (JP)
(74) Representative: Albrecht, Thomas, Dr.
(86) International application number: PCT/JP2002/002174
(87) International publication number: WO 2002/072041

(57) **Abstract**

Skin care cosmetics or dermatological preparations which comprise, as the active ingredient, moisturizer(s), antioxidant(s) or mineral salt(s) which are substances having an efficacy to promote maturation of cornified envelopes.

## Description

### Technical Field

This invention belongs to the technical field of cosmetics or dermatological preparations. More specifically, the invention relates to cosmetics containing an agent to promote formation or maturation of cornified envelope (hereinafter abbreviated as CE) or use of the promoting agent in dermatological field.

### Background Art

The stratum corneum is composed of corneocytes formed of terminal differentiation of keratinocytes of the epidermis and intercellular lipids surrounding the same. It has now become clear that the intercellular lipids take a lamella structure with its components such as ceramides, cholesterol, fatty acids and the like, to play an important role in the barrier function of the stratum corneum. This is verified by the fact that intercellular lipids exhibit both morphotype and constitutional disorder in various skin diseases resulting in deterioration in the barrier function of the stratum corneum and such skin troubles as rough skin. Whereas, corneocytes are composed mainly of keratin fibers and of CE (cornified envelope) enveloping the corneocytes. The CE is formed as the plural CE precursor proteins, which are produced at keratinocytes of the epidermis accompanying differentiation of said cells, are crosslinked by enzymic transglutaminase and insolubilized. It is furthermore suggested: certain ceramides covalently bind to a part of the CE to form a hydrophobic construction, whereby providing the basis for aforesaid lamella structure of intercellular lipids, which are indispensable for the barrier function of the stratum corneum.

CE can be prepared through the steps of boiling epidermal tissue or cultured keratinocytes in a solution containing a surfactant such as sodium dodecylsulfate and a reducing agent such as mercaptoethanol and recovering the insoluble fraction by removing the soluble components by such means as centrifugation. Microscopic observation of form of so obtained CE enables evaluation of its properties. Michel, et al. reported: compared to the outermost layer of stratum corneum, at deep part of the stratum corneum many CE's have fragile structure (J. Invest. Dermatol. 91: 11-15, 1988). Furthermore, they state fragile CE's are found also in the outermost layer in patients suffering from psoriasis and keratoderma (Br. J. Dermatol., 122: 15-21, 1990).

We have engaged in investigations with the view to diagnose skin, in particular, conditions of the stratum corneum, based on the CE properties, to discover that abnormality in the barrier function of the stratum corneum could be grasped by examining hydrophobic property of CE by, for example, dyeing with Nile Red and also antigenicity of involucrin which is a constituent of CE, by immunostaining, in addition to the form observation.

We also discovered that the utilization of such dyeability enables evaluation of formation or maturation of CE in cultured keratinocytes. Based on such discoveries, we proposed in the past an evaluation method of skin quality (JP 2001-91514A).

On the other hand, in treating skin diseases like psoriasis wherein fragile CE's are observed even at the outermost stratum corneum and which is accompanied by parakeratosis (or deterioration in the barrier function of the stratum corneum) as observed in pathologic tissue, mainly external remedies such as corticosteroid have been used. Also various countermeasures have been proposed to cope with skin troubles such as rough skin which are attributable to deterioration in the barrier function of the stratum corneum.

As above, although various means have been proposed to cope with parakeratosis of skin (or immaturity of keratinocytes) or deterioration in the barrier function of the stratum corneum, necessity for providing still more effective means does exist. In particular, means for accelerating formation or maturation of CE both quantitatively and qualitatively is in demand, as CE has been suggested to play the key role in the barrier function of the stratum corneum.

### Disclosures of the Invention

The skin quality evaluation method which we have developed detected many immature CE's in the stratum corneum on, for example, the faces of subjects exhibiting deteriorated skin barrier function. We noticed the possibility that the immature CE's might be retaining the ability to mature, and carried out further investigations to discover that the immature CE's could be converted to mature CE's under prescribed conditions. We furthermore discovered that continuous use of skin care cosmetics, in which prescribed active component satisfying said conditions are blended, promotes CE formation in the human skin and improves the barrier function of the skin. The present invention is completed based on this knowledge.

Accordingly, therefore, this invention relates to skin care cosmetics comprising as the active component at least one substance exhibiting efficacy for promoting maturation of cornified envelope (CE), which substance being selected from the group consisting of moisturizers, antioxidants and mineral salts.

Another embodiment of the present invention relates to use of at least one substance exhibiting efficacy for promoting maturation of cornified envelope (CE), which is (are) selected from the group consisting of moisturizers, antioxidants and mineral salts, for the purpose of formulating preparations exhibiting the efficacy for improving the skin affected by parakeratosis or skin with stratum corneum showing deteriorated barrier function.

Still another embodiment of the present invention concerns a method for treating the skin affected by parakeratosis or the skin with stratum corneum showing deteriorated barrier function, said method comprising administering onto the human skin requiring a treatment a substance or substances exhibiting the efficacy for promoting maturation of cornified envelope (CE) in the skin, of an amount sufficient to promote maturation of CE, said substance being at least one member selected from the group consisting of moisturizers, antioxidants and mineral salts.

According to the present invention, thus improvement or treatment of the skin suffering from dermatological diseases can be accomplished by skin care following ordinary use of cosmetics.

### Best Mode for Carrying Out the Invention

"Formation or maturation of CE" as referred to in the invention signifies the process in which plural CE precursor proteins, which are produced with differentiation of epidermal keratinocytes, are crosslinked by enzyme transglutaminase and insobubilized, and furthermore certain ceramides are covalently bonded with a part of such insolubilized matter to form hydrophobic structure. Therefore, formation or maturation of CE is detectable by positive staining with a dye capable of selectively dyeing hydrophobic regions, such as Nile Red, or reduction or disappearance in antigenicity of CE-constituting proteins (more specifically, reduction or disappearance of the binding ability of the antibodies with the said proteins).

Exhibition of "efficacy for promoting maturation of CE" according to the present invention signifies exhibition of an action and effect to bring about above-described formation or maturation of CE, in actual uses of cosmetics following the present invention. Hence, according to the invention the specific active component is used in such an embodiment as will bring about the formation or maturation of CE.

As examples of the active component, moisturizer selected from polyols and derivatives thereof, natural polysaccharides and derivatives thereof, hydrophilic synthetic polymers and betaines can be named. The polyols and derivatives thereof are the compounds having at least two hydroxyl groups per molecule and their derivatives, e.g., monosaccharide, oligosaccharide, sugar alcohol, ethylene oxide adducts and the like. As specific examples of such polyols, while not limited thereto, glycerin, 1,3-propanediol, 2-methyl-1,3-propanediol, 1,4-butanediol, 1,3-butanediol, 1,5-pentanediol, diglycerin, erythritol, gluconic acid, 1,2,6-hexanetriol, inositol, lactitol, maltitol, mannitol, xylitol and trehalose can be named. As natural polysaccharides and derivatives thereof, plant-or microorganism-derived polysaccharides and derivatives thereof, e.g., hyaluronic acid, acetylhyaluronic acid, heparin, polyglucuronic acid, chondroitin, chondroitin sulfate, xanthane gum, pectin and, if acceptable, alkali metal salts of the foregoing can be used. As hydrophilic synthetic polymers, poly(2-methacryloyloxyethyl-phosphorylcholine) and poly(2-methacryloyloxyethylphosphoryl-CO-methacrylamide) can be named.

Useful bataines are betaine (which also is occasionally referred to as Glycine-betaine) and derivatives thereof, and any of the compounds falling within the scope of ampho-ionic compounds of corresponding sultaine (in which carboxyl of betaine is replaced with sulfo) so long as it meets the purpose of the invention. Those representative of such derivatives are polymers in which 1-3 N-methyl groups in betaine are replaced with other optionally branched, saturated or unsaturated hydrocarbon chain(s); or such hydrocarbon chain(s) which is (are) interrupted by amide linkage of the following formula,

- [CₙH₂ₙCONH(CH₂)ₘ]-

(in which m and n stand for an integer of 1-30 independently of each other);
or the hydrocarbon chain length between the quaternary ammonium group and carboxyl or sulfo group varies (e.g., number of the carbon atoms varies between 2-6); or the polymers having plural betaine residues as pendant groups. They may be cyclic compounds. Specific examples of such derivatives include, while not to be construed as being limited thereto, besides those named in the above, γ-butyrobetaine, decylbetaine, laurylbetaine, myristylbetaine, cetylbetaine, stearylbetaine, behenylbetaine, lauramidopropylbetaine, oleamidopropylbetaine, palmitamidopropylbetaine, γ-butyrobetaine, laurylsultine, cocosultaine, poly(metharyloyloxyethylbetaine), poly(methacryloyloxyethylbetaine-co-2-hydroxyethylmethacrylic acid) and the like.

As antioxidants which are useful in the present invention, ascorbic acid and salts or derivatives thereof, sulfur-containing organic compounds, in particular, those which can be present in either reduced form or oxidized form; and tocopherol and derivatives thereof can be named. Examples of such antioxidants include ascorbic acid; magnesium ascorbate, sodium ascorbate and the like; and ascorbic acid derivatives such as alkyl esters, phosphoric acid esters, sulfuric acid esters and glucoside of ascorbic acid. More specifically, examples of ascorbic acid alkyl ester include ascorbyl palmitate, ascorbyl diisomyristate, ascorbyl 2-ethylhexanoate, ascorbyl di-2-ethylhexanoate, ascorbyl aleate and ascorbyl dioleate. As other ascorbic acid derivatives, polypeptide ascorbate, ascorbyl magnesium phosphate, methylsilanol ascorbate, ascorbyl tocopherylpotassium phosphate and the like can be named.

On the other hand, tocopherol and derivatives thereof can also be used, examples of the derivatives including, while not limited thereto, tocopherol esters such as tocopheryl acetate, tocopheryl linoleate, tocopheryl nicotinate and tocopheryl succinate; and ethylene oxide adducts of tocopherol such as tocophereth-5, tocophereth-10 and tocophereth-12. Also sulfur-containing organic compounds (including derivatives of sulfur-containing amino acid) and their metabolic intermediates such as acetyl cysteine, methionine, cysteine, homocysteine, glutathione, hypotaurine, cysteine-sulfinic acid, cysteic acid, thiocysteine, taurine, thiotaurine, djenkolic acid, cystathionine, S-allylcysteine, renthionine, ethionine, dithiothreitol, thioglycerin and α-lipoic acid can be used.

Furthermore, as mineral salts useful for the invention, hydrohalogenic acid salts of calcium, magnesium or zinc, in particular, calcium chloride, magnesium chloride and zinc chloride; salts with phosphoric acid, in particular, calcium phosphate and calcium dihydrogenphosphate; and salts with sulfuric acid, in particular, calcium sulfate and zinc sulfate can be named.

More than one of such components within a same class or of different classes as above-described can be used as mixed or in combination.

As preferred examples of such combined use, mixtures of moisturizer(s) or antioxidant(s) with the mineral salt(s) can be named.

The above-described substance(s) are compounded as active component of skin care cosmetics following the present invention, generally in an amount ranging 0.001-20% by weight, preferably 0.1-10% by weight, per the total weight of the cosmetic or a dermatological preparation. Thus compounded cosmetic can be formulated by mixing with, or homogenized in, diluent or carrier which are acceptable for preparation of cosmetics or dermatological preparations, for example, pure water or ion-exchange water or buffered water; lower alkanol such as methanol, ethanol or isopropyl alcohol or hydrous solutions thereof, or oil component such as hardened castor oil, vaseline, squalane and the like, if necessary with concurrent use of surfactant or the like. Into cosmetics or dermatological preparations of the present invention, still other components customarily used for external applications like cosmetics and drugs, such as whitening agent, oil component, ultraviolet absorber, surfactant, higher alcohol, powder component, colorant, aqueous component, water, various skin tonics or the like can be suitably blended, within the range not detrimental to the effect of the present invention. The compositions according to the present invention may further suitably comprise sequestering agent such as disodium edetate, trisodium edetate, sodium citrate, sodium polyphosphate, sodium metaphosphate, gluconic acid and the like; caffeine, tannine, verapamil, tranexamic acid and derivatives thereof, glabridin; hot water extract of Chinese quince, crude drugs other than the above; drug such as glycyrrhetinic acid, derivatives or salts thereof, whitening agent such as kojic acid; and vitamin A derivatives such as retinoic acid, retinol, retinol acetate, retinol palmitate and the like; may be suitably blended.

Forms of those cosmetics or dermatological preparations according to the present invention are not critical and they can be used in optional formulation forms such as solution, solubilized, emulsified, powderized, water-oil bilayer, water-oil-powder trilayer, ointment, gel and airsol systems. Their form of use may also be optional, for example, facial cosmetics and foundations such as lotion, liquid preparation, milky lotion, cream, packs and the like; and also as make-up cosmetics, cosmetics for hair, fragrant cosmetics, bath salts and the like, with the understanding that their uses are not limited to the above. Such cosmetics or preparations normally are applied to the skin on the desired part to exhibit the efficacy of promoting maturation of CE as above explained, or in an amount sufficient to bring about said efficacy. Such an amount can be determined by experts of cosmetology or dermatology after examination of individual cases and consideration of preliminary use results.

Hereinafter the present invention is explained more specifically referring to Examples which are given for the purpose of illustration only.

### Example 1: CE Maturation Promoting Test

Stratum corneum containing many immature CE's were taken from the subjects suffering from skin troubles such as chapping, by means of tape stripping. To the stratum corneum still adhering to the tapes, a test solution was applied, followed by 4 days' incubation under the conditions of 30°C in temperature and 70% in humidity. After end of the incubation, a tris-hydrochloric acid buffer solution containing dithiothreitol and sodium dodecylsulfate was added, followed by 10 minutes' heating at 100°C. The insoluble matter was collected by centrifuge at 4000 g for 10 minutes. Addition of the eluate and heating cycle was further repeated to thoroughly remove the soluble component. Thus obtained insoluble matter was used as CE.

Properties of each CE thus isolated were evaluated by the following method: CE was spotted on a piece of slide glass, air-dried and fixed in cold acetone. Hydrating the same with Dulbecco's phosphate buffered saline, it was reacted with mouse anti-human involucrin antibody (NOVOCASTRA Co.) as the primary antibody. Excessive antibody was removed by washing and the residue was reacted with FITC-labeled rabbit anti-mouse immunoglobulin antibody as the secondary antibody. Excessive antibody was removed by washing, and the residue was reacted with Nile Red dye solution, sealed and observed with a fluorescence microscope. The observed images were taken into computer via CCD camera, and the ratio of involucrin positive immature CE and that of Nile Red-positive mature CE were evaluated using an image analysis software (Win Roof) or the like. The results were as shown in Table 1.

**Table 1**

| CE Maturation Promoting Effect | |
|---|---|
| Applied Test Solution | Ratio of Immature CE (%) |
| water | 11.7 |
| 10% glycerin | 6.3 |
| 8% glycerin + 1.3% erythritol | 1.9 |
| Mean of N = 3 | |

### Examples 2 and 3:

Procedures similar to Example 1 were repeated, except that the incubation conditions were varied to 37°C and 100% (relative humidity) for a day. The results of using various test solutions were as shown in the following Tables 2 and 3.

**Table 2**

| CE Maturation Promoting Effect | |
|---|---|
| Applied Test Solution | Ratio of Immature CE (%) |
| water | 36.9 |
| 10% glycerin | 14.6 |
| 10% trimethyl glycine | 9.3 |
| Mean of N = 3 | |

**Table 3**

| CE Maturation Promoting Effect | |
|---|---|
| Applied Test Solution | Ratio of Immature CE (%) |
| before treatment | 28.3 |
| 0.1 M MES buffer pH5.5 | 3.0 |
| 10 mM dithiothreitol | 0.33 |
| 1 mM calcium chloride | 1.83 |
| 10 mM dithiothreitol + 1 mM calcium chloride | 0.19 |
| Mean of N = 3 | |

### Example 4: CE Maturation Promoting Effect by Continuous Use of Skin Care Cosmetics

Female volunteers suffering from rough skin were let to use skin care series cosmetics containing glycerin and erythritol continuously for 4 weeks. Their stratum corneum samples were collected before and after said test period, and the CE evaluation was conducted following the method as described in Example 1. The ratio of immature CEs was determined by visual evaluation in 5-rating (0,1,2,3 and 4) and expressed as immature CE score. As shown in Table 4, the results verified reduction in the ratio of immature CE after use of the cosmetics. The effect of the continued use of these skin care cosmetics to promote maturation of CE could be thus confirmed.

**Table 4**

| CE Maturation Promoting Effect by Continued Use of Skin Care Cosmetics | |
|---|---|
| Stratum Corneum Sample | Ratio of Immature CE (score) |
| before use | 1.65 |
| after use | 1.04 |
| Mean of N = 13 | |

| Preparation Example 1: Milk Lotion | |
|---|---|
| (Formula) | % by weight |
| stearic acid | 2.5 |
| cetyl alcohol | 1.5 |
| vaseline | 5.0 |
| liquid paraffin | 5.0 |
| polyoxyethylene (10) monooleate | 2.0 |
| polyethylene glycol 1500 | 3.0 |
| triethanolamine | 1.0 |
| carboxyvinyl polymer | 0.05 |
| glycerin | 5.0 |
| erythritol | 1.0 |
| sodium hydrogensulfite | 0.01 |
| ethylparaben | 0.2 |
| perfume | q.s. |
| ion-exchange water | balance |

### (Formulation method)

The carboxyvinyl polymer was dissolved in a minor amount of the ion-exchange water (A phase). The polyethylene glycol 1500, triethanolamine, glycerin and erythritol were added to the rest of the ion-exchange water, dissolved under heating and maintained at 70°C (aqueous phase). All other ingredients were mixed, melted under heating and maintained at 70°C (oil phase). The oil phase was added to the aqueous phase to conduct preliminary emulsification, into which A phase was added and homogeneously emulsified with a homogenizing mixer. The formulation was cooled to 30°C after the emulsification under thorough mixing, to provide a milk lotion.

| Preparation Example 2: Lotion | |
|---|---|
| (Formula) | % by weight |
| ethanol | 5.0 |
| carboxyvinyl polymer | 0.3 |
| polyoxyethylene (15) oleyl ether | 0.8 |
| glycerin | 5.0 |
| 1,3-butylene glycol | 5.0 |
| thiotaurine | 0.1 |
| citric acid | 0.03 |
| sodium citrate | 0.07 |
| calcium chloride | 0.1 |
| methylparaben | 0.1 |
| ion-exchange water | balance |

### (Preparation method)

The citric acid, sodium citrate and calcium chloride were dissolved in the ion-exchange water, to provide the aqueous phase. Separately, other ingredients were stirred and dissolved, and the solution was added to the aqueous phase and homogenized to provide a lotion.

| Preparation Example 3: Cream | |
|---|---|
| (Formula) | % by weight |
| stearic acid | 2.0 |
| stearyl alcohol | 7.0 |
| hydrogenated lanolin | 2.0 |
| 2-octyldodecyl alcohol | 6.0 |
| polyoxyethylene (25) cetyl alcohol ether | 3.0 |
| stearic acid monoglyceride | 2.0 |
| 1,3-butylene glycol | 5.0 |
| trisodium edetate | 0.1 |
| sodium glycyrrhetinate | 0.1 |
| erythritol | 2.0 |
| α-lipoic acid | 0.1 |
| vitamin E acetate | 0.3 |
| ethylparaben | 0.3 |
| ion-exchange water | balance |

### (Preparation method)

To the ion-exchange water, 1,3-butylene glycol, trisodium edetate, sodium glycyrrhetinate, erythritol and α-lipoic acid were added and kept at 70°C, which served as the aqueous phase. Separately, all other ingredients were mixed, melt under heating and kept at 70°C, which served as the oil phase. The oil phase was added to the aqueous phase, preliminarily emulsified, homogeneously emulsified with a homogenizing mixer and cooled to 30°C to provide a cream.

### Industrial Applicability

According to the invention, an effective means for ameliorating rough skin caused by immaturity of CE at the skin or for treating skin diseases is provided. Accordingly, the present invention is utilizable in industries relating to cosmetics and dermatology.

## Claims

1. Skin care cosmetics comprising an effective amount of at least one substance selected from moisturizer, antioxidant and mineral salt, which has the efficacy to promote maturation of cornified envelope (CE) at the skin, and diluent or carrier which are acceptable for preparation of cosmetics or dermatologically acceptable.

2. Skin care cosmetics according to Claim 1, in which the moisturizer is selected from polyols and derivatives thereof, natural polysaccharides and derivatives thereof, hydrophilic synthetic polymers and betaines.

3. Skin care cosmetics according to Claim 1, in which the antioxidant is selected from ascorbic acid, salts or derivatives thereof, sulfur-containing organic compounds; and tocopherol and derivatives thereof.

4. Skin care cosmetics according to Claim 1, in which the mineral salt is a salt of a metal selected from calcium, magnesium and zinc.

5. Skin care cosmetics according to Claim 1, which comprises a mixture of moisturizer(s) or antioxidant(s) with mineral salt(s).

6. Use of at least one substance selected from the group consisting of moisturizer, antioxidant and mineral salt for formulating preparations which have an efficacy to improve the skin affected by parakeratosis or the skin with stratum corneum showing deteriorated barrier function, said substance having an efficacy for promoting maturation of cornified envelopes (CE).

7. The use according to Claim 6, in which the moisturizer is selected from polyols and derivatives thereof, natural polysaccharides and derivatives thereof, hydrophilic synthetic polymers and betaines; the antioxidant is selected from ascorbic acid and salts or derivatives thereof, sulfur-containing organic compounds and tocopherol and derivatives thereof, and the mineral salt is selected from calcium, magnesium and zinc.

8. The use according to Claim 6, in which the substance having the efficacy to promote maturation of CE is a mixture of the moisturizer(s) or antioxidant(s) with the mineral salt(s).

9. A method of treating the skin affected by parakeratosis or the skin with stratum corneum showing deteriorated barrier function, which method comprises administering to the human skin whose treatment is required, at least one substance which is selected from moisturizer, antioxidant and mineral salt and which has an efficacy to promote maturation of cornified envelopes (CE) at the skin, in an amount sufficient to promote maturation of CE.

10. The treating method according to Claim 9, in which the moisturizer is selected from polyols and derivatives thereof, natural polysaccharides and derivatives thereof, hydrophilic synthetic polymers and betaines; the antioxidant is selected from ascorbic acid and salts or derivatives thereof, sulfur-containing organic compounds and tocopherol and derivatives thereof, and the mineral salt is selected from calcium, magnesium and zinc.

11. The treating method according to Claim 9, in which the substance having the efficacy to promote maturation of CE is a mixture of the moisturizer(s) or antioxidant(s) with the mineral salt(s).
